# EUROPEAN PATENT APPLICATION

(11) **EP 4 130 284 A1**
(43) Date of publication of application: **08.02.2023**
(21) Application number: 21775110.6
(22) Date of filing: 22.03.2021
(51) Int. Cl.: C12P 7/08, B09B 3/00, C02F 11/10, C02F 11/13, C07C 29/152, C07C 31/08, C10J 3/00, C10J 3/72, C12M 1/00, C12M 1/107, C12P 7/06

(54) **PRODUCTION METHOD FOR ORGANIC SUBSTANCE AND ORGANIC SUBSTANCE PRODUCTION DEVICE**

(30) Priority: 24.03.2020 JP 2020052811; 31.03.2020 JP 2020064478
(71) Applicant: SEKISUI CHEMICAL CO., LTD., Osaka-shi Osaka 530-8565 (JP)
(72) Inventor: MIKI, Nanami, Tokyo 105-8566 (JP); HAMACHI, Kokoro, Tokyo 105-8566 (JP); NATSUYAMA, Kazuto, Tokyo 105-8566 (JP)
(74) Representative: Ter Meer Steinmeister & Partner
(86) International application number: PCT/JP2021/011803
(87) International publication number: WO 2021/193572

(57) **Abstract**

The production method for an organic substance comprises: a step of feeding waste (G0) to a dryer (13); a step of drying the waste (G0) by the dryer (13); a step of feeding the waste (G0) dried by the dryer (13) to a gasifier (14); a step of gasifying the waste (G0) by the gasifier (14) to generate synthetic gas (G1); and a step of bringing the synthetic gas (G1) into contact with a microbial catalyst to generate an organic substance.

## Description

### Technical Field

The present invention relates to a production method for an organic substance, the method being for producing an organic substance from synthetic gas as a raw material, and an organic substance production device that is for producing the organic substance from synthetic gas as a raw material.

### Background Art

Techniques to obtain synthetic gas from various types of waste, including industrial waste and domestic waste, are widely known. In these techniques, gas is generated through pyrolysis in a gasification furnace and the generated gas is then reformed in a reforming furnace. The resulting synthetic gas is directly combusted for use in power generation or other purpose, or is subjected to heat recovery, as necessary, with a boiler or the like and then used for power production or other purpose. Attempts to use synthetic gas as a raw material for chemical synthesis have been made in recent years; for example, conversion of synthetic gas into an organic substance such as ethanol by using a microbial catalyst has been attempted (e.g., see PTL1).

PTL2 discloses a method for obtaining synthetic gas from a biosolid such as dehydrated sludge. In this method, a biosolid having a solid content of 30% by mass or less is mixed with soot or tar, the mixture is dried with a dryer until a solid content of 75% by mass or more is reached, and the mixture after being dried is subjected to partial oxidation to give synthetic gas.

### Citation List

### Patent Literature

PTL1: JP 2019-167424 A
PTL2: US 2014/0158940 A1

### Summary of Invention

### Technical Problem

In general, waste is collected from various sites and gathered in a wastedisposal facility or the like, temporarily stored in a storage unit such as a waste pit, and then loaded into a gasification furnace or the like. Being highly uneven in terms of components, the waste stored in the storage unit at that time is typically mixed in advance with a crane or the like before loading into a gasification furnace.

However, such waste is still highly uneven even after being mixed with a crane or the like; for example, the moisture content of garbage may be highly uneven. For this reason, even if waste is loaded in equal portions into a gasification furnace by using a dust feeder or the like, the amounts of hydrogen and carbon monoxide obtained per unit time may have large variation. For example, in using synthetic gas for power generation, the electric energy varies according to the variation of the generations of hydrogen and carbon monoxide; however, the hydrogen and carbon monoxide generated are mostly available, thus allowing efficient power generation to be achieved.

In generating an organic substance such as ethanol from synthetic gas as a raw material, for example, by using a microbial catalyst, shortage of feed rates of hydrogen and carbon monoxide due to the variation of the generations of them may kill the microbial catalyst. Thus, in using waste-derived synthetic gas, it is necessary to reduce the amount of a microbial catalyst so as not to cause shortage of hydrogen and carbon monoxide fed to the microbial catalyst in view of the variation of the generations of hydrogen and carbon monoxide. Hence, it is difficult to efficiently generate an organic substance such as ethanol by using waste-derived synthetic gas as a raw material.

Moreover, like PTL2, techniques for obtaining synthetic gas are known in which the moisture contents of raw materials of synthetic gas are reduced below specific levels by drying them; however, drying the whole waste with a dryer causes a problem of high energy loads.

In addition, for drying waste with a dryer, the dryer needs maintenance on regular basis because of pollution caused by waste, which makes long-term continuous operation difficult, thus complicating feeding synthetic gas for a long period of time without interruption. On the other hand, in generating an organic substance such as ethanol from synthetic gas as a raw material by using a microbial catalyst, feeding of synthetic gas to the microorganism without interruption is required so as not to kill the microorganism. For these reasons, incorporating the step of drying waste makes it difficult to achieve implementation of generation of an organic substance by using a microbial catalyst.

In view of such circumstances, an object of the present invention is to provide a production method for an organic substance and an organic substance production device that allow an organic substance such as ethanol to be efficiently generated from synthetic gas as a raw material by using a microbial catalyst with reduced variation of the generations of hydrogen and carbon monoxide in obtaining synthetic gas.

Another object of the present invention is to provide a production method for an organic substance and an organic substance production device that allow an organic substance such as ethanol to be generated from synthetic gas as a raw material in an efficient manner for a long period of time without interruption.

### Solution to Problem

The summary of the present invention is as shown in the following [1] to [33].
[1] A production method for an organic substance, the production method comprising:
   a step of feeding waste to a dryer;
   a step of drying the waste by the dryer;
   a step of feeding the waste dried by the dryer to a gasifier;
   a step of gasifying the waste by the gasifier to generate synthetic gas; and
   a step of bringing the synthetic gas into contact with a catalyst to generate an organic substance.
[2] The production method for an organic substance according to [1], the production method further comprising:
   a step of receiving waste in a first storage unit, wherein
   the waste stored in the first storage unit is fed to the dryer.
[3] The production method for an organic substance according to [1] or [2], the production method comprising:
   a step of feeding waste dried by the dryer to a second storage unit, wherein
   the waste stored in the second storage unit is fed to the gasifier.
[4] The production method for an organic substance according to [3], the production method comprising:
   a step of mixing the waste stored in the second storage unit.
[5] The production method for an organic substance according to any one of [1] to [4], wherein the waste is dried to reach a moisture content of 5% by mass or more and 30% by mass or less and fed to the gasifier.
[6] The production method for an organic substance according to any one of [1] to [5], the production method comprising:
   a step of measuring the moisture content of at least one of the waste before being dried by the dryer and the waste dried by the dryer.
[7] The production method for an organic substance according to any one of [1] to [5], the production method further comprising:
   a step of measuring the moisture content of waste, wherein
   waste whose determined moisture content is equal to or higher than a threshold is fed to the dryer.
[8] The production method for an organic substance according to [7], wherein waste whose determined moisture content is lower than the threshold is fed to the gasifier without passing through the dryer.
[9] The production method for an organic substance according to [7] or [8], wherein the threshold is set within a range of 10 to 35% by mass.
[10] The production method for an organic substance according to any one of [7] to [9], the production method comprising:
   a step of feeding waste whose determined moisture content is lower than the threshold to a second storage unit to store the waste in the second storage unit; and
   a step of feeding the waste stored in the second storage unit to the gasifier.
[11] The production method for an organic substance according to any one of [6] to [10], wherein waste stored in a first storage unit is taken out and the moisture content of the waste is measured.
[12] The production method for an organic substance according to [11], wherein the waste stored in the first storage unit is taken out by a crane and held by the crane and the moisture content of the waste held is measured.
[13] The production method for an organic substance according to [12], wherein the crane transfers waste whose determined moisture content is equal to or higher than a threshold to feed to the dryer, and transfers waste whose determined moisture content is lower than the threshold to feed to the gasifier without passing through the dryer.
[14] The production method for an organic substance according to any one of [6] to [13], wherein a moisture-measuring device configured to measure the moisture content of waste comprises a protective instrument or a washing instrument.
[15] The production method for an organic substance according to any one of [1] to [14], wherein the organic substance comprises ethanol.
[16] The production method for an organic substance according to any one of [1] to [15], wherein the catalyst is a microbial catalyst.
[17] An organic substance production device, comprising:
   a dryer configured to dry waste;
   a gasifier configured to gasify waste to generate synthetic gas; and
   an organic substance generation unit configured to bring the synthetic gas into contact with a catalyst to generate an organic substance, wherein
   the organic substance production device is capable of feeding waste dried by the dryer to the gasifier.
[18] The organic substance production device according to [17], further comprising:
   a first storage unit configured to receive waste; and
   a waste feeder capable of feeding waste stored in the first storage unit to the dryer and capable of feeding waste dried by the dryer to the gasifier.
[19] The organic substance production device according to [18], further comprising:
   a second storage unit, wherein
   the waste feeder is capable of feeding waste dried by the dryer to the second storage unit and capable of feeding waste stored in the second storage unit to the gasifier.
[20] The organic substance production device according to [19], wherein
   the waste feeder is capable of mixing waste stored in the second storage unit, or
   the organic substance production device further comprises a mixer configured to mix waste stored in the second storage unit.
[21] The organic substance production device according to any one of [18] to [20], wherein
   the waste feeder feeds waste dried to reach a moisture content of 5% by mass or more and 30% by mass or less to the gasifier.
[22] The organic substance production device according to any one of [17] to [21], comprising:
   a moisture-measuring device configured to measure the moisture content of at least one of waste before being dried by the dryer and waste dried by the dryer.
[23] The organic substance production device according to any one of [17] to [21], further comprising:
   a moisture-measuring device configured to measure the moisture content of waste, wherein
   the organic substance production device is capable of feeding waste whose moisture content has been determined to the dryer.
[24] The organic substance production device according to [23], being capable of feeding waste whose moisture content has been determined to the gasifier without passing through the dryer.
[25] The organic substance production device according to [23] or [24], wherein waste whose determined moisture content is equal to or higher than a threshold is fed to the dryer, and waste whose determined moisture content is lower than the threshold is fed to the gasifier without passing through the dryer.
[26] The organic substance production device according to [25], wherein the threshold is set within a range of 10 to 35% by mass.
[27] The organic substance production device according to any one of [23] to [26], further comprising:
   a second storage unit configured to be fed with waste whose moisture content has been determined and store the waste, wherein
   the organic substance production device is capable of feeding waste stored in the second storage unit to the gasifier.
[28] The organic substance production device according to any one of [22] to [27], comprising:
   a first storage unit configured to store waste; and
   a take-out device configured to take out waste stored in the first storage unit, wherein
   the moisture-measuring device measures the moisture content of waste taken out by the take-out device.
[29] The organic substance production device according to [28], wherein
   the take-out device is a crane, and
   the moisture-measuring device measures the moisture content of waste take out and held by the crane.
[30] The organic substance production device according to [29], wherein
   the crane is capable of transferring waste whose moisture content has been determined to feed to the dryer or feed to the gasifier without passing through the dryer.
[31] The organic substance production device according to any one of [22] to [30], wherein
   the moisture-measuring device comprises a protective instrument or a washing instrument.
[32] The organic substance production device according to any one of [17] to [31], wherein the organic substance comprises ethanol.
[33] The organic substance production device according to any one of [17] to [32], wherein the catalyst is a microbial catalyst.

### Advantageous Effects of Invention

The present invention enables efficient generation of an organic substance such as ethanol from synthetic gas as a raw material by using a catalyst such as a microbial catalyst with reduced variation of the generations of hydrogen and carbon monoxide in obtaining synthetic gas.

Additionally, in the present invention, the moisture content of waste is measured, and waste whose determined moisture content is equal to or higher than a threshold is fed to a dryer and dried. Thus, the present invention can provide a production method for an organic substance and an organic substance production device that allow an organic substance such as ethanol to be efficiently generated from synthetic gas as a raw material for a long period of time without interruption by using a catalyst such as a microbial catalyst.

### Brief Description of Drawings

[Fig. 1] Fig. 1 shows a schematic diagram illustrating the entire configuration of an organic substance production device according to a first embodiment of the present invention.
[Fig. 2] Fig. 2 shows a schematic graph for explaining the operation and effect of the present invention.
[Fig. 3] Fig. 3 shows a schematic diagram illustrating the entire configuration of an organic substance production device according to a second embodiment of the present invention.
[Fig. 4] Fig. 4 shows a schematic diagram illustrating the entire configuration of an organic substance production device according to a third embodiment of the present invention.
[Fig. 5] Fig. 5 shows a schematic diagram illustrating a feed stream to feed waste from a first storage unit to a gasifier via a dryer (Fig. 5(A)) and a feed stream to feed waste from the first storage unit to a hopper via a second storage unit (Fig. 5(B)) in the third embodiment.
[Fig. 6] Fig. 6 shows a schematic diagram illustrating the entire configuration of an organic substance production device according to a fourth embodiment of the present invention.
[Fig. 7] Fig. 5 shows a schematic diagram illustrating a feed stream to feed waste from a first storage unit to a gasifier via a dryer (Fig. 7(A)) and a feed stream to feed waste from the first storage unit to the gasifier via a second dust feeder (Fig. 7(B)) in the fourth embodiment.

### Description of Embodiments

### <First Embodiment>

Now, the present invention will be described by way of embodiments with reference to the drawings.

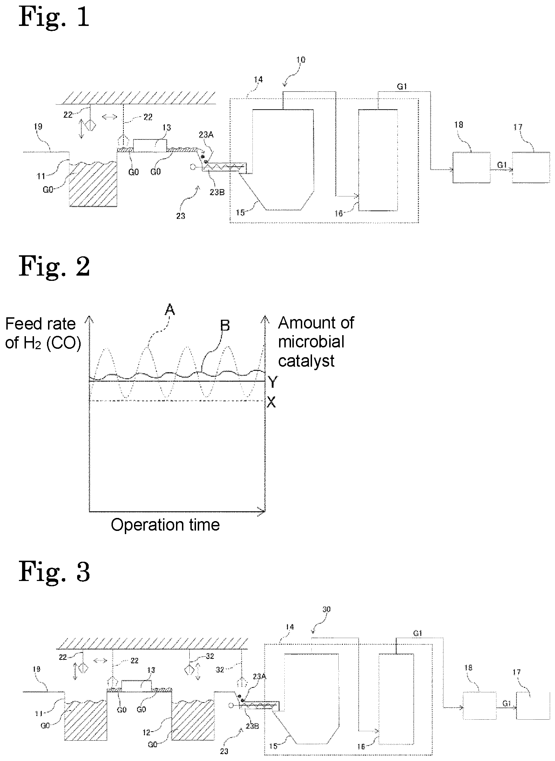

Fig. 1 shows an organic substance production device 10 according to the first embodiment of the present invention. The organic substance production device and production method for an organic substance according to the first embodiment will now be described in detail with reference to Fig. 1.

As illustrated in Fig. 1, the organic substance production device 10 includes: a storage unit (occasionally referred to as the "first storage unit") 11; a dryer 13; a gasifier 14; and an organic substance generation unit 17.

### (Storage Unit)

The storage unit 11, which is a unit configured to receive and store waste G0, is a waste pit, for example. The waste G0 may be industrial waste such as industrial solid waste, or domestic waste such as municipal solid waste (MSW), and examples of the waste G0 include combustibles including plastic waste, garbage, waste tires, biomass waste, food waste, building materials, woods, wood chips, fibers, and waste paper. Among them, municipal solid waste (MSW) is preferred. The waste G0 generally contains a certain level of moisture, and domestic waste such as municipal solid waste (MSW) contains about 20 to 60% by mass of moisture, more typically, contains about 30 to 50% by mass of moisture.

For example, the storage unit 11 (hereinafter, occasionally referred to as the "first storage unit") receives the waste G0 from a platform 19 provided adjacently to the storage unit 11. For example, the platform 19 can be set such that a garbage truck is allowed to stop thereon and load the waste G0 into the storage unit 11; however, the method for loading of the waste G0 is not limited thereto.

A crane 22 as a waste feeder is provided above the storage unit 11. The crane 22 is movable, for example, in the horizontal and vertical directions, and capable of holding the waste G0 and releasing the waste G0 held. In this way, the crane 22 can feed the waste G0 stored in the storage unit 11 to the dryer 13. As specifically described later for the third embodiment, the crane 22 normally includes a holding section and a hanging section.

Further, the crane 22 is capable of mixing the waste G0 within the storage unit 11 by moving the waste G0 stored in the storage unit 11 within the storage unit 11, or by repeatedly holding the waste G0 and releasing the waste G0 held. Mixing the waste G0 in the storage unit 11 facilitates reduction of the unevenness of the moisture content of the waste G0.

Mixing of the waste G0 in the storage unit 11 may be performed by using a mixer other than the crane 22 such as a stirring blade.

### (Dryer)

The dryer 13 dries the waste G0 fed from the storage unit 11. The mode of the dryer 13 is not limited, and the dryer 13 may be a batch dryer or a conveyor dryer.

A conveyor dryer is an apparatus configured to dry the waste G0 in a continuous manner while the waste G0 is moved from a loading inlet to a discharge outlet. A conveyor dryer is shown as a representative example of the dryer 13 in Fig. 1; however, the dryer 13 is not limited thereto.

Examples of the conveyor dryer include a rotary dryer and a conveyor-belt dryer. The rotary dryer rotates a cylindrical rotational shell to dry and simultaneously move the waste G0 loaded into the shell. The conveyor-belt dryer dries the waste G0 within the dryer while a conveyor belt conveys the waste G0.

The batch dryer, which is an apparatus configured to dry the waste G0 batch by batch, heats the loaded waste G0 within the dryer for a given period of time and then takes out the waste G0, thereby drying the waste G0.

The drying mode of the dryer 13 is not limited, and may be either direct drying, in which the waste G0 is dried by allowing hot air to flow through the inside of the dryer, or indirect drying, which achieves heating through heat transfer in heating an inner surface of the apparatus in contact with the waste G0 (e.g., for the rotary dryer, the inner surface of the rotational shell); alternatively, another mode may be used. In the indirect drying, the inner surface of the apparatus can be heated with a heating medium passing through a heat transfer tube provided within the apparatus. Examples of the heating medium include steam, but are not limited thereto.

The temperature within the dryer 13 in drying the waste G0 (drying temperature) can be set so that the post-drying moisture content of the waste G0 can fall within a specific range described later, and is, for example, 50 to 400°C, preferably 100 to 300°C. The waste G0 can be dried at the above-described drying temperature, for example, for 1 to 10 minutes, preferably for 2 to 8 minutes.

The organic substance production device 10 further includes a dust feeder 23 as a waste feeder. The dust feeder 23 feeds the waste G0 dried by the dryer 13 to the gasifier 14. For example, the dust feeder 23 includes a hopper 23A and a feed screw 23B, and moves the waste G0 loaded into the hopper 23A by rotating the feed screw 23B, thereby feeding the waste G0 to a gasification furnace 15.

As described above, the waste G0 is dried in advance by the dryer 13, and fed to the gasifier 14 by the dust feeder 23. The waste G0, in particular, domestic waste such as municipal solid waste (MSW), generally has high unevenness in moisture.

The moisture content of the waste G0 decreases if the waste G0 is dried by the dryer 13, and the dryer 13 is normally capable of reducing the moisture content below a specific level (e.g., 30% by mass or less) with ease by virtue of the drying ability, irrespective of whether the moisture contained in the waste G0 before drying is much or not; however, it is difficult for the dryer 13 to further reduce the moisture content below a much lower specific level (e.g., 5% by mass or less). Accordingly, the unevenness of the moisture content of the waste G0 can be reduced through drying of the waste G0 by the dryer 13.

The waste G0 can be dried by the dryer 13, for example, to reach a moisture content of 5% by mass or more and 30% by mass or less, and the waste G0 dried to reach such a moisture content can be fed to the gasifier 14. When the moisture content of the waste G0 is 5% by mass or more, the spontaneous ignition of the waste G0 is prevented, and the dryer 13 does not need to have excessively high drying ability.

When the moisture content is 30% by mass or less, the unevenness of the post-drying moisture content of the waste G0 can be kept within a specific range with ease. In addition, the energy consumption for evaporating the moisture in the gasifier 14 described later can be reduced.

From those viewpoints, the moisture content is preferably 10% by mass or more and 25% by mass or less, and more preferably 15% by mass or more and 20% by mass or less. To reach the moisture content of the waste G0 within the range, the drying conditions for the dryer can be tailored to the type of the waste G0.

Although the crane 22 has been shown hereinabove as a waste feeder to feed the waste G0 stored in the storage unit 11 to the dryer 13, a device other than the crane 22 may be used; for example, a conveyor whose power source is electricity, gas such as air and nitrogen, or steam, such as a conveyor belt, a dust feeder, and a hopper, may be used. Needless to say, the waste feeder to feed the waste G0 to the dryer 13 may be a combination of a crane and a conveyor other than cranes, or a combination of two or more conveyors other than cranes.

Likewise, the waste feeder to feed the waste G0 dried by the dryer 13 to the gasifier 14 is not limited to the dust feeder 23, and a conveyor other than dust feeders may be used, including those whose power source is electricity, gas such as air and nitrogen, or steam, such as a conveyor belt, a crane, and a hopper. A combination of a dust feeder and a conveyor other than dust feeders may be used, and a combination of two or more conveyors other than dust feeders may be used.

### (Gasifier)

The gasifier 14 gasifies the waste to generate synthetic gas. The gasifier 14 includes a gasification furnace 15 and a reforming furnace 16.

Examples of the gasification furnace 15 include, but are not limited to, a kiln gasification furnace, a fixed-bed gasification furnace, a fluidized-bed gasification furnace, a plasma gasification furnace (plasma method), a shaft furnace (shaft method), and a thermoselect furnace (thermoselect method). Another example thereof is a carbonization furnace. In addition to the waste, oxygen or air are loaded into the gasification furnace 15, and water vapor is further loaded thereinto, as necessary. The gasification furnace 15 pyrolyzes the waste G0 by heating, for example, at 500 to 700°C to appropriately cause partial oxidation, thereby gasifying the waste G0. The pyrolysis gas contains not only carbon monoxide and hydrogen, but also gaseous tar, powdery char, and others. The pyrolysis gas is fed to the reforming furnace 16. Solid matters formed as incombustibles in the gasification furnace 15 can be collected as appropriate.

In the reforming furnace 16, the pyrolysis gas given by the gasification furnace 15 is reformed to give synthetic gas. At least one of the contents of hydrogen and carbon monoxide in the pyrolysis gas increases in the reforming furnace 16, and the resultant is discharged as synthetic gas G1. In the reforming furnace 16, for example, tar, char, and others contained in the pyrolysis gas are converted into hydrogen, carbon monoxide, etc., through reforming.

The temperature of the synthetic gas in the reforming furnace 16 is not limited, and, for example, 900°C or higher, preferably 900°C or higher or 1300°C or lower, and more preferably 1000°C or higher and 1200°C or lower, and the synthetic gas can be discharged out of the reforming furnace 16 (i.e., the gasifier 14) at such a temperature. When the temperature in the reforming furnace 16 is within the range, synthetic gas having high contents of carbon monoxide and hydrogen can be obtained with ease.

The synthetic gas G1 discharged from the reforming furnace 16 (i.e., the gasifier 14) contains carbon monoxide and hydrogen. The synthetic gas G1 contains, for example, 0.1% by volume or more and 80% by volume or less of carbon monoxide, and 0.1% by volume or more and 80% by volume or less of hydrogen.

The carbon monoxide concentration of the synthetic gas G1 is preferably 10% by volume or more and 70% by volume or less, and more preferably 20% by volume or more and 55% by volume or less. The hydrogen concentration of the synthetic gas G1 is preferably 10% by volume or more and 70% by volume or less, and more preferably 20% by volume or more and 55% by volume or less.

The synthetic gas G1 may contain carbon dioxide, nitrogen, oxygen, and others in addition to hydrogen and carbon monoxide. The carbon dioxide concentration of the synthetic gas G1 is not limited, but preferably 0.1% by volume or more and 40% by volume or less, and more preferably 0.3% by volume or more and 30% by volume or less. It is particularly preferable in performing ethanol generation with a microbial catalyst that the carbon dioxide concentration be low, and from such a viewpoint the carbon dioxide concentration is more preferably 0.5% by volume or more and 25% by volume or less.

The nitrogen concentration of the synthetic gas G1 is typically 60% by volume or less, may be 40% by volume or less, and is preferably 1% by volume or more and 20% by volume or less.

The oxygen concentration of the synthetic gas G1 is typically 5% by volume or less, and preferably 1% by volume or less. The oxygen concentration is preferably as low as possible, and can be 0% by volume or more. However, oxygen is inevitably contained in general, and the oxygen concentration is practically 0.01% by volume or more.

The carbon monoxide, carbon dioxide, hydrogen, nitrogen, and oxygen concentrations of the synthetic gas G1 can be set within specific ranges by appropriately modifying the conditions for combustion, including the type of the waste, the post-drying moisture content of the waste G0, the temperatures of the gasification furnace 15 and the reforming furnace 16, and the oxygen concentration of the feed gas to be fed to the gasification furnace 15. If the carbon monoxide and hydrogen concentrations are to be changed, the waste is replaced with waste having a high proportion of hydrocarbon (carbon and hydrogen) such as plastic waste; if the nitrogen concentration is to be lowered, for example, a method of feeding gas having a high oxygen concentration to the gasification furnace 15 is contemplated.

Further, the synthetic gas G1 may be subjected to concentration adjustment for the components, specifically, carbon monoxide, carbon dioxide, hydrogen, and nitrogen, as appropriate. The concentration adjustment can be performed by adding at least one of the components to the synthetic gas G1.

It should be noted that % by volume of each substance in the synthetic gas G1 shown above means % by volume of each substance in the synthetic gas G1 discharged from the gasifier 14.

Although a mode in which the gasifier 14 includes the gasification furnace 15 and the reforming furnace 16 has been shown hereinabove, the configuration of the gasifier 14 is not limited thereto, and the gasifier 14 may be an apparatus in which a gasification furnace and a reforming furnace are integrated together, and may be a gasifier of any mode that can generate the synthetic gas G1.

The synthetic gas G1 given by the gasifier 14 is sent to the organic substance generation unit 17. As illustrated in Fig. 1, the synthetic gas G1 is normally sent to the organic substance generation unit 17 after being appropriately treated in a post-stage treatment device 18. In the post-stage treatment device 18, impurities contained in the synthetic gas G1 are removed and the synthetic gas G1 is cooled, as appropriate; for example, the synthetic gas G1 can be cooled to 40°C or lower and fed to the organic substance generation unit 17. The details of the post-stage treatment device 18 are described later.

### (Organic Substance Generation Unit)

The organic substance generation unit 17 generates an organic substance by bringing the synthetic gas into contact with a microbial catalyst. A gas-assimilating microorganism is preferably used as a microbial catalyst. The organic substance generation unit 17 includes a fermentation tank (reactor) filled with culture solution containing water and a microbial catalyst. The synthetic gas G1 is fed into the fermentation tank, and converted into an organic substance within the fermentation tank. The organic substance may be an alcohol such as isopropanol, methanol, and ethanol, or isoprene, or a hydrocarbon that is available as a raw material of jet fuel, for example. The organic substance preferably contains ethanol or isopropanol, and more preferably contains ethanol.

The fermentation tank is preferably a continuous bioreactor, and may be any of a stirring bioreactor, an airlift bioreactor, a bubble-column bioreactor, a loop bioreactor, an open-bond bioreactor, and photobioreactor.

Although the synthetic gas G1 and culture solution may be fed to the fermentation tank in a continuous manner, it is not needed to feed the synthetic gas G1 and culture solution at the same time, and the synthetic gas G1 may be fed to the fermentation tank to which culture solution has been fed in advance. The synthetic gas G1 is typically blown into the fermentation tank via a sparger or the like.

The medium for use in culturing the microbial catalyst is a liquid containing water as a main component and nutrients dissolved or dispersed in the water (e.g., vitamins, phosphate), and the medium is not limited as long as it is a medium having a proper composition for the microorganism.

In the organic substance generation unit 17, an organic substance is generated through the microbial fermentation by the microbial catalyst, and an organic substance solution results.

The fermentation tank is preferably controlled to keep the temperature at 40°C or lower. The control to keep the temperature at 40°C or lower prevents the microbial catalyst in the fermentation tank from being killed, allowing an organic substance such as ethanol to be efficiently generated through the contact between the synthetic gas and the microbial catalyst.

The temperature of the fermentation tank is more preferably 38°C or lower; for enhanced catalytic activity, the temperature of the fermentation tank is preferably 10°C or higher, more preferably 20°C or higher, and even more preferably 30°C or higher.

In the present embodiment, as described above, the waste G0 is dried so that the moisture content can fall within a specific range, and converted into the synthetic gas G1 in the gasifier 14. By virtue of this, as shown in Fig. 2, the variation of feed rates of hydrogen and carbon monoxide to the organic substance generation unit 17 per unit time in the case that the waste G0 is dried, B, is lower than the variation of those in the case that the waste G0 is not dried, A.

Therefore, if drying is avoided, it is necessary in view of the variation caused by the avoidance to decrease the amount of the microbial catalyst, X, in the fermentation tank so as not to kill the microbial catalyst because of the occurrence of shortage of hydrogen and carbon monoxide fed to the fermentation tank. If the waste G0 is dried, on the other hand, the variation of feed rates of hydrogen and carbon monoxide, B, is lower, and hence the amount of the microbial catalyst can be increased from X to Y, as shown in Fig. 2. Accordingly, the amounts of hydrogen and carbon monoxide unused in generation of an organic substance can be decreased to increase the generation of an organic substance per unit time in the organic substance generation unit 17. Thus, the present embodiment allows an organic substance such as ethanol to be efficiently generated by using the microbial catalyst.

### (Separator)

The organic substance production device 10 may include a separator (not shown) configured to separate at least water from the organic substance solution.

Examples of the separator include a solid-liquid separator, a distillation device, and a separation membrane, and use of a solid-liquid separator and a distillation device in combination is preferred. A separation step performed by using a solid-liquid separator and a distillation device in combination will be specifically described below.

It should be noted that the separator may be omitted, for example, if the organic substance produced in the organic substance generation unit 17 does not need purification or if separation of water from the organic substance solution is not needed.

In the solid-liquid separator, the organic substance solution given in the organic substance generation unit 17 can be separated into a solid component primarily containing the microorganism and a liquid component containing the organic substance. Since the organic substance solution given in the organic substance generation unit 17 contains not only the organic substance as the target product, but the microorganism contained in the fermentation tank and dead cells or the like thereof as the solid component, solid-liquid separation is performed to remove the solid component. Examples of the solid-liquid separator include a filter, a centrifuge, and a device using a technique of solution precipitation. The solid-liquid separator may be a device configured to evaporate the liquid component containing the organic substance from the organic substance solution to separate the liquid component from the solid component (e.g., a heat dryer). At that time, the liquid component containing the organic substance as the target product may be completely evaporated, or partially evaporated in such a manner that the target organic substance preferentially evaporates.

The liquid component separated through the solid-liquid separation can be further subjected to distillation to separate the organic substance as the target product in the distillation device. The separation by distillation allows the organic substance to be massively purified with high purity by simple operations.

In performing distillation, a known distillation device such as a distillation column can be used. In distillation, operations can be performed so that the distillate can contain the organic substance (e.g., ethanol) as the target product with high purity and the bottom solution (i.e., the distillation residue) can contain water as a primary component (e.g., 70% by mass or more, preferably 90% by mass or more). Such operations allow the organic substance as the target product and water to be almost completely separated from each other.

The temperature in the distillator in the distillation of the organic substance (e.g., ethanol or isopropanol, in particular, ethanol) is not limited, but preferably 100°C or lower, and more preferably about 70 to 95°C. Setting the temperature in the distillation device within the above-described range ensures separation of the organic substance as the requisite from other components including water.

The pressure in the distillation device in the distillation of the organic substance may be normal pressure, but is preferably lower than the atmospheric pressure, and more preferably about 60 to 150 kPa (gauge pressure). The separation efficiency for the organic substance can be enhanced and the yield of the organic substance can be enhanced as well by setting the pressure in the distillation device within the above-described range.

The water separated in the separator is preferably reused, and, for example, can be fed to a gas-cooling tower of the post-stage treatment device 18 described later and used for water spray in the gas-cooling tower.

### (Post-stage treatment device)

Examples of the post-stage treatment device 18 include a heat exchanger, a gas-cooling tower, a filter dust collector, a water scrubber, an oil scrubber, a moisture separator including a gas chiller or the like, a separator employing lowtemperature separation (cryogenic separator), a microparticle separator including different filters, a desulfurizer (sulfide separator), a separator employing membrane separation, a deoxidizer, a pressure swing adsorption separator (PSA), a temperature swing adsorption separator (TSA), a pressure-temperature swing adsorption separator (PTSA), a separator using activated carbon, a separator using a deoxidizing catalyst, specifically, a copper catalyst or palladium catalyst, and a processor like a shift reactor. One of these processors may be used singly, and two or more thereof may be used in combination.

Among them, at least a heat exchanger, a gas-cooling tower, a filter dust collector, and a water scrubber are preferably included in the post-stage treatment device 18 in the presented order from the pre-stage.

The term "pre-stage" herein means the pre-stage in the feed stream of the waste G0 and the synthetic gas G1 generated. The term "post-stage" means the post-stage in the feed stream of the waste G0 and the synthetic gas G1. The feed stream of the waste G0 and the synthetic gas G1 in the first and second embodiments refers to a series of flows of the waste G0 and the synthetic gas G1: from the feeding of the waste G0 from the storage unit 11 to the dryer 13, through the subsequent generation of the synthetic gas G1 in the gasifier 14, to the introduction of the synthetic gas G1 into the organic substance generation unit 17. The feed stream of the waste G0 and the synthetic gas G1 in the third and fourth embodiments described later refers to a series of flows of the waste G0 and the synthetic gas G1: from feeding of the waste G0 from a first storage unit 111 to a gasifier 114 via a dryer 113 or not via the dryer 113, through the subsequent generation of the synthetic gas G1 in the gasifier 114, to the introduction of the synthetic gas G1 into an organic substance generation unit 117.

The heat exchanger, a device configured to cool the synthetic gas G1 with a heating medium, cools the synthetic gas G1 by transferring the thermal energy of the synthetic gas G1 to the heating medium. A boiler is preferably used as the heat exchanger. A boiler is a device configured to circulate water as a heating medium therein and heat the circulated water to convert into steam by the thermal energy of the synthetic gas G1. Use of a boiler as the heat exchanger allows other units to be heated with ease by steam generated by the heat exchanger, and the thermal energy of the synthetic gas G1 can be reused with ease. Needless to say, a device other than boilers may be used as the heat exchanger.

While the temperature of the synthetic gas G1 within the gasifier 14 is high and likewise the synthetic gas discharged from the gasifier 14 has a high temperature, for example, of 900°C or higher as described above, the synthetic gas G1 having a relatively low temperature resulting from cooling by the heat exchanger is fed to the gas-cooling tower in the post-stage; thereby, excessive cooling in the gas-cooling tower can be prevented.

The heat exchanger can cool the synthetic gas G1 fed at a high temperature, for example, of 900°C or higher to reach a temperature, for example, of 200°C or higher and 300°C or lower, preferably of 240°C or higher and 280°C or lower, and feed the synthetic gas G1 to the gas-cooling tower.

The synthetic gas G1 discharged from the heat exchanger can be further passed through the gas-cooling tower; the synthetic gas G1 is further cooled by passing through the gas-cooling tower.

In the gas-cooling tower, the synthetic gas G1, for example, introduced from the upper part and passed through the inside as downward gas flow is cooled by water sprayed from a water-spraying port provided in the inner surface of the cooling tower during passing through the inside. The synthetic gas G1 cooled in the gas-cooling tower can be discharged from the lower part of the gas-cooling tower.

While the synthetic gas G1 to be introduced to the gas-cooling tower has a temperature sufficiently higher than 100°C, the water sprayed from the water-spraying port has a temperature lower than 100°C. Thus, the synthetic gas G1 is cooled because of the temperature difference, and additionally cooled because of the heat of vaporization required for the vaporization of the water sprayed from the water-spraying port. Part of the vaporized water can be mixed as water vapor into the synthetic gas G1. The water sprayed from the water-spraying port may have been completely or partially vaporized when being sprayed.

After being cooled to a temperature preferably of 100°C or higher and 200°C or lower, more preferably of 120°C or higher and 180°C or lower, even more preferably of 130°C or higher and 170°C or lower in the gas-cooling tower, the synthetic gas G1 cooled to such a temperature can be discharged out of the gas-cooling tower.

Cooling the synthetic gas to 200°C or lower enables purification of the synthetic gas using the filter dust collector described later without damaging the filter dust collector or deteriorating the dust collection performance thereof. The temperature being 100°C or higher causes most of the water sprayed to evaporate and thus mix in the synthetic gas. As a result, massive discharge of sprayed water is avoided in the gas-cooling tower, and hence there is no need to introduce largescaled discharge equipment for the gas-cooling tower.

The filter dust collector, for which what is called a bug filter can be used, removes solid impurities such as tar and char by passing therethrough the synthetic gas cooled in the gas-cooling tower. The removing of solid impurities allows units in the post-stage of the filter dust collector to be prevented from being clogged with solid impurities.

In the present specification, "remove" means that the concentration of a target substance to be removed in the gas is reduced by removing at least a part of the target substance from the synthesis gas and is not limited to the complete removing of the target substance to be removed.

The synthetic gas after passing through the filter dust collector can be further passed through the water scrubber. The water scrubber removes impurities contained in the synthetic gas by bringing the synthetic gas passing through the inside of the scrubber into contact with water. The water scrubber removes water-soluble impurities including acidic gases such as hydrogen sulfide, hydrogen chloride, and hydrocyanic acid, basic gasses such as ammonia, and oxides such as NOx and SOx. In addition, oily impurities and others such as BTEX (benzene, toluene, ethylbenzene, xylene), naphthalene, 1-naphtol, and 2-naphtol may be removed, as appropriate.

The water scrubber is not limited and may be any water scrubber having such a configuration that the synthetic gas G1 and water are brought into contact with each other; for example, the water scrubber can have such a configuration that water (also referred to as "washing water" for convenience in the following) sprayed from a nozzle provided in the upper part is brought into contact with the synthetic gas G1 passing through the inside of the water scrubber from the lower part to the upper part.

The water scrubber can cool the synthetic gas G1 by bringing the synthetic gas G1 into contact with washing water. The synthetic gas G1 is cooled to a specific temperature in the gas-cooling tower and introduced at the temperature to the water scrubber, as described above. On the other hand, the temperature of the washing water to be brought into contact with the synthetic gas in the water scrubber is lower than 100°C, preferably 0°C or higher and 40°C or lower, and more preferably 5°C or higher and 30°C or lower.

Through contact with water at the above-described temperature in the water scrubber, the synthetic gas G1 is cooled to a temperature, for example, of lower than 100°C, preferably of 40°C or lower, more preferably of 38°C or lower. The synthetic gas G1 can be cooled, for example, to a temperature of 0°C or higher, and preferably cooled to a temperature of 5°C or higher through contact with the washing water in the water scrubber. The synthetic gas G1 cooled to 40°C or lower does not kill the microbial catalyst even when being fed to the organic substance generation unit 17 with the temperature retained.

Further, by passing through the water scrubber, the synthetic gas G1 can be removed of water mixed therein in the gas-cooling tower while the synthetic gas G1 is washed and cooled in the water scrubber.

The synthetic gas discharged from the water scrubber may be appropriately passed through one or more of the above processors other than the heat exchanger, the gas-cooling tower, the filter dust collector, and the water scrubber for purification, cooling, or any other processing of the synthetic gas.

Although a configuration in which the heat exchanger, the gas-cooling tower, the filter dust collector, and the water scrubber are all provided in the post-stage of the gasifier 14 has been described hereinabove, some or all of them may be omitted. Even if the water scrubber is omitted, for example, another cooling device can be provided in the post-stage to cool the synthetic gas G1 to 40°C or lower and feed the synthetic gas G1 to the organic substance generation unit 17. At least one of the heat exchanger, the gas-cooling tower, and the filter dust collector may also be omitted, and purification, cooling, or any other processing of the synthetic gas can be performed only with processors other than the heat exchanger, the gas-cooling tower, the filter dust collector, and the water scrubber.

### <Second Embodiment>

Next, the organic substance production device and production method for an organic substance according to the second embodiment of the present invention will described in detail. An organic substance production device 30 according to the second embodiment differs from that according to the first embodiment in that a second storage unit 12 is included, as a storage unit, in addition to a storage unit (first storage unit) 11. The second embodiment will now be described with reference to Fig. 3. In the following description, a component having the same configuration as the corresponding component in the first embodiment is provided with the same reference sign, and description thereof is omitted.

In the present embodiment, the second storage unit 12, which is a unit configured to store waste dried by the dryer 13, is a waste pit, for example. The second storage unit 12 is disposed in the post-stage of the dryer 13 and fed with the waste G0 dried by the dryer 13, and stores the waste G0 after being dried. The waste G0 stored in the second storage unit 12 can be then fed to the gasifier 14.

A crane 32 (second crane) as a waste feeder is provided above the second storage unit 12. The crane 32 is movable, for example, in the horizontal and vertical directions, and capable of holding the waste and releasing the waste held. In this way, the crane 32 can feed the waste dried by the dryer 13 and discharged from the dryer 13 to the second storage unit 12. Further, the crane 32 is capable of moving the waste G0 stored in the second storage unit 12, for example, can feed the waste G0 to the hopper 23A of the dust feeder 23, and can feed the waste G0 in the second storage unit 12 after being dried to the gasifier 14 via the dust feeder 23.

Furthermore, the crane 32 is capable of mixing the waste G0 within the second storage unit 12 by moving the waste G0 stored in the second storage unit 12 within the second storage unit 12, or by repeatedly holding the waste G0 and releasing the waste G0 held. Alternatively, mixing of the waste G0 in the second storage unit 12 may be performed by using a mixer other than the crane 32 such as a stirring blade. The waste G0 within the second storage unit 12 can be fed to the gasifier 14 after being mixed.

The weight of the waste G0 stored in the second storage unit 12 is lower than that before drying because the waste G0 has been dried, and the reduced moisture content allows the waste G0 to be prevented from sticking, thus facilitating mixing of the waste G0 stored in the second storage unit 12. Accordingly, the waste G0 stored in the second storage unit 12 can be homogenized through being mixed by the crane 32, and the unevenness of the moisture content of the waste to be fed to the gasifier 14 can be more reduced. In addition, the unevenness of just the components constituting the waste G0 can be reduced. Accordingly, the variation of feed rates of carbon monoxide and hydrogen to the organic substance generation unit 17 per unit time can be more reduced, which allows more efficient generation of the organic substance.

A combination of the crane 32 and the dust feeder 23 exemplifies a waste feeder to feed the waste G0 stored in the second storage unit 12 to the gasifier 14 in the second embodiment, but a combination differing from the mentioned one may be used to feed the waste G0 stored in the second storage unit 12 to the gasifier 14. For example, the crane 32 alone or the dust feeder 23 alone may be used, and a device other than cranes and dust feeders may be used; for example, a conveyor whose power source is electricity, gas such as air and nitrogen, or steam, such as a conveyor belt and a hopper, may be used. Needless to say, two or more of such conveyors may be used in combination.

Likewise, a device other than the crane 32 is also applicable as a waste feeder to feed the waste G0 dried by the dryer 13 to the second storage unit 12; for example, a conveyor other than cranes may be used, including those whose power source is electricity, gas such as air and nitrogen, or steam, such as a conveyor belt, a dust feeder, and a hopper. Needless to say, the waste feeder to feed the waste G0 dried by the dryer 13 to the second storage unit 12 may be a combination of two or more conveyors.

Although the first storage unit 11 and the second storage unit 12 are disposed at positions distant from each other and provided with the cranes (the first and second cranes) 22 and 32, respectively, in Fig. 3, the first storage unit 11 and the second storage unit 12 may be disposed at adjacent positions.

If the first and second storage units 11 and 12 are disposed at adjacent positions, it is not needed to provide a crane to each storage unit, and conveyance and mixing of the waste G0 in the first storage unit 11 and those in the second storage unit 12 may be performed with one crane.

### <Modifications of First and Second Embodiments>

The thus-described organic substance production device and production method for an organic substance as illustrated with the first and second embodiments are an example of the present invention, and the present invention is not limited to the configurations of those embodiments; any improvement and modification can be made without departing from the spirit of the present invention, and another component may be added as appropriate.

For example, each of the organic substance production devices 10 and 30 according to those embodiments may include a moisture analyzer (also referred to as a "moisture-measuring device") configured to measure the moisture content of the waste G0. A known moisture meter can be used as the moisture analyzer.

The moisture meter is not limited and may be any moisture meter that can measure the moisture content of the waste G0; applicable are moisture meters listed later as moisture-measuring devices. Needless to say, the moisture meter may include at least one of a protective instrument and a washing instrument, each described later. The moisture content of the waste G may be determined from a measurement at one point, and the mean for a plurality of points may be used as the moisture content of the waste.

The moisture analyzer can be, for example, a post-drying moisture analyzer configured to measure the post-drying moisture content of the waste G0, which has been dried by the dryer 13. Whether the moisture content of the waste G0 after being dried has reached a target moisture content can be determined by measuring the post-drying moisture content of the waste G0. The target moisture content may be, for example, one value in the range of 5 to 30% by mass, and any target moisture content within a specific range (e.g., 5 to 30% by mass, 10 to 25% by mass, or 15 to 20% by mass) can be employed in normal cases.

If the moisture content has not reached the target moisture content, the moisture content can be adjusted to reach the target moisture content, for example, by appropriately modifying the conditions for drying in the dryer 13 (drying temperature, drying time, etc.).

The post-drying moisture analyzer can measure the moisture content of the waste G0, for example, from immediately after being discharged from the dryer 13 to immediately before being loaded into the gasifier 14; for example, the moisture content of the waste G0 to be fed to the dust feeder 23 may be measured, and the moisture content of the waste G0 immediately after being discharged from the dryer 13 may be measured.

In the second embodiment, the moisture content of the waste G0 stored in the second storage unit 12 may be measured. The unevenness of the moisture content of the waste G0 stored in the second storage unit 12 can be reduced, for example, by mixing by the crane 32 or a mixer other than the crane 32, as described above. Thus, the post-drying moisture content of the waste G0 can be accurately clarified by measuring the moisture content of the waste G0 stored in the second storage unit 12.

The moisture analyzer is not limited to the thus-described post-drying moisture analyzer, and may be a pre-drying moisture analyzer configured to measure the moisture content of the waste G0 before being dried by the dryer 13.

The pre-drying moisture analyzer can measure the moisture content of the waste G0 from after being loaded into the first storage unit 11 to before being loaded into the dryer 13; for example, the moisture content of the waste G0 stored in the first storage unit 11 may be measured, and the moisture content of the waste G0 immediately before loading into the dryer 13 may be measured.

On the basis of measurement of the pre-drying moisture content of the waste G0 by using the pre-drying moisture analyzer in the described manner, the moisture content can be controlled to reach the target moisture content, for example, by appropriately modifying the conditions for drying (drying temperature, drying time, etc.) according to the moisture content determined.

Needless to say, both the pre-drying moisture analyzer and the post-drying moisture analyzer may be provided to control the moisture content to reach the target moisture content, for example, by appropriately modifying the conditions for drying according to measurements from the two. If both the pre-drying moisture analyzer and the post-drying moisture analyzer are provided, the moisture content of the waste G0 can be more precisely adjusted, and hence the variation of feed rates of hydrogen and carbon monoxide to be fed to the organic substance generation unit 17 is more reduced.

Although the catalyst for generating an organic substance is a microbial catalyst in the first and second embodiments, the catalyst is not limited to the microbial catalyst, and may be a metal catalyst as described later in detail for the third and fourth embodiments. In the first and second embodiments, the first storage unit 11 may be omitted.

Next, preferred embodiments of the organic substance production device including a moisture-measuring device, and a production method for an organic substance, the method including a step of measuring the moisture content of waste, will be described in more detail by way of the following third and fourth embodiments.

### <Third Embodiment>

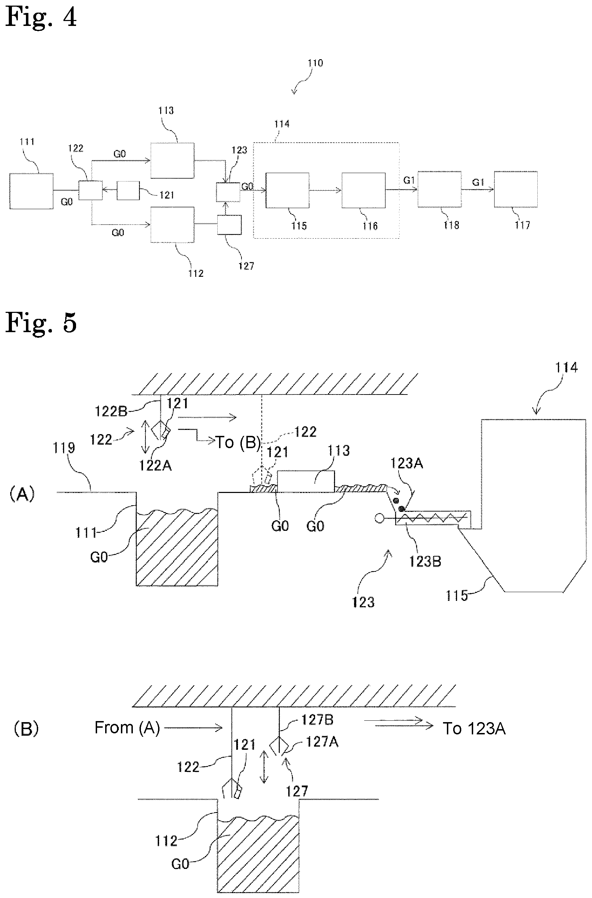

Fig. 4 shows a block diagram illustrating an organic substance production device according to the third embodiment of the present invention. Fig. 5 shows a schematic diagram illustrating in detail a part of the organic substance production device 110 according to the third embodiment. The organic substance production device and production method for an organic substance according to the third embodiment will now be described in detail with reference to Figs. 4 and 5.

As illustrated in Fig. 4, the organic substance production device 110 includes: a first storage unit 111; a second storage unit 112; a dryer 113; a gasifier 114; an organic substance generation unit 117; and a moisture-measuring device 121.

The first storage unit 111, which is a unit configured to receive and store waste G0, is a waste pit, for example. The waste G0 is as described for the first embodiment and description thereof is omitted; the waste G0 is preferably municipal solid waste (MSW) also in the present embodiment. The waste G0 generally contains a certain level of moisture, and domestic waste such as municipal solid waste (MSW) contains about 20 to 60% by mass of moisture, more typically, contains about 30 to 50% by mass of moisture.

In the present embodiment, however, it is also preferable that the first storage unit 111 receive not only MSW but also other waste, for example, a wide variety of combustibles including plastic waste, garbage, waste tires, biomass waste, food waste, building materials, woods, wood chips, fibers, and waste paper. Different types of waste typically have different moisture contents, and in the present embodiment, waste having a high moisture content is dried to reduce the unevenness of the moisture content before being fed to the gasifier 114, as described later.

As illustrated in Fig. 5, for example, the first storage unit 111 receives the waste G0 from a platform 119 provided adjacently to the first storage unit 11 in the same manner as in the first embodiment.

The organic substance production device 110 can include a crane 122, and the crane 122 (hereinafter, also referred to as the first crane 122) is a take-out device to be used for taking out the waste in the first storage unit 111. The first crane 122 is provided above the first storage unit 111 as illustrated in Fig. 5.

The first crane 122 includes a holding section 122A for holding the waste G0 and a hanging section 122B configured to hang the holding section 122A and move the holding section 122A in the vertical direction. For example, the hanging section 122B is hung from the ceiling, being movable in the horizontal direction along the ceiling. In this way, the first crane 122 is capable of holding the waste G0 with the holding section 122A and releasing the waste G0 held, and further capable of moving the waste G0 held in the horizontal and vertical directions.

For example, the first crane 122 is capable of transferring the waste G0 from the first storage unit 111 to the vicinity of the dryer 113 (see Fig. 5(A)). In addition, the first crane 122 is capable of transferring the waste G0 from the first storage unit 111 to the second storage unit 112 (see Fig. 5(B)). Thus, the crane 122 can take out the waste G0 stored in the first storage unit 111 from the first storage unit 111 and feed to the dryer 113 or the second storage unit 112. In other words, the crane 122 also constitutes a feeder for feeding the waste to the dryer 113 or the second storage unit 112 (waste feeder).

Further, the first crane 122 is capable of mixing the waste G0 within the first storage unit 111 by moving the waste G0 stored in the first storage unit 111 within the first storage unit 111 or by repeatedly holding the waste G0 and releasing the waste G0 held. Mixing the waste G0 in the first storage unit 111 facilitates reduction of the unevenness of the moisture content of the waste G0. Alternatively, mixing of the waste G0 in the first storage unit 111 may be performed by using a mixer other than the crane 122 such as a stirring blade.

### (Second Storage Unit)

The second storage unit 112, which is a unit configured to receive and store the waste G0, is a waste pit, for example. The second storage unit 112 receives and stores the waste G0 transferred by the crane 122 from the first storage unit 111 and subjected to measurement of the moisture content as described later. To the second storage unit 112 in the present embodiment, the waste G0 is fed without passing through the dryer 113 described later; thus the waste G0 not dried by the dryer 113 is stored in the second storage unit 112. It should be noted that, as described later, the waste G0 to be fed to the second storage unit 112 is waste whose moisture content has been determined to be lower than a threshold X in measurement by the moisture-measuring device 121, and hence the waste G0 having a low moisture content is stored in the second storage unit 112.

The second storage unit 112 can be used, for example, as a spare feed source to feed the waste G0 to the gasifier 114 when the waste G0 cannot be fed from the dryer 113 because of, for example, maintenance of the dryer 113.

A second crane 127 can be provided above the second storage unit 112. As with the case of the first crane 122, the second crane 127 includes a holding section 127A and a hanging section 127B. The second crane 127 is capable of holding the waste G0 with the holding section 127A and releasing the waste G0 held, and movable in the horizontal and vertical directions.

The waste G0 stored in the second storage unit 112 can be fed to a hopper 123A of the dust feeder 123 described later by the second crane 127, and can be fed to the gasifier 114 via the dust feeder 123.

The waste G0 stored in the second storage unit 112 can be mixed either by the first crane 122 or by the second crane 127. Alternatively, mixing of the waste G0 in the second storage unit 112 may be performed by using a mixer other than cranes such as a stirring blade.

### (Dryer)

The dryer 113 is an apparatus configured to dry the waste G0. The dryer 113 dries the waste G0 fed from the first storage unit 111 by the first crane 122. The details of the configuration of the dryer 113 are as described for the first embodiment. A conveyor dryer is shown as a representative example of the dryer 113 in Fig. 5; however, the dryer 113 is not limited thereto.

The temperature within the dryer 113 in drying the waste G0 (drying temperature) can be set so that the post-drying moisture content of the waste G0 can fall within a specific range described later, and is, for example, 50 to 400°C, preferably 100 to 300°C. The waste G0 can be dried at the above-described drying temperature, for example, for 1 to 10 minutes, preferably for 2 to 8 minutes. When the drying temperature and the drying time are within the above-described ranges, the waste G0 having a moisture content equal to or higher than a threshold X as described later can be sufficiently dried, and it is easy to achieve a moisture content, for example, of lower than a threshold X.

The waste G0 transferred from the first crane 122 may be fed to the dryer 113 via an additional dust feeder (not shown) or the like.

A storage unit for the dryer (not shown) may be appropriately provided in the pre-stage of the dryer 113 (but in the post-stage of the first storage unit 111). If the storage unit for the dryer is provided, a specific volume of the waste G0 transferred by the crane 122 for feeding to the dryer 113 can be temporarily stored therein, which facilitates drying of the waste G0 with the above-described batch dryer. Moreover, the operation of the dryer 113 can be readily suspended for maintenance for some time.

The storage unit for the dryer may be, for example, a waste pit, or a hopper or the like that serves as a loading inlet for the waste may be used as the storage unit for the dryer if the waste is fed to the dryer 113 via a dust feeder (not shown).

### (Dust Feeder)

As illustrated in Fig. 5, the organic substance production device 110 can further include a dust feeder 123 (hereinafter, occasionally referred to as the first dust feeder 123) as a feeder (waste feeder). The dust feeder 123 feeds the waste G0 dried by the dryer 113 and the waste G0 stored in the second storage unit 112 to the gasifier 114. As with the case of the dust feeder 23 in the above-described first embodiment, the dust feeder 123 includes, for example, a hopper 123A and a feed screw 123B as illustrated in Fig. 5(A), and feeds the waste G0 loaded into the hopper 123A to a gasification furnace 115. The waste G0 is fed to the gasifier 114 via the dust feeder 123; as a result, the waste G0 is constantly fed.

As described above, the waste G0 is fed from both the dryer 113 and the second storage unit 112 to the gasifier 114 via the dust feeder 123; however, any method may be employed for the procedure of feeding.

For example, while the waste G0 is fed from the dryer 113, the feeding of the waste G0 from the second storage unit 112 can be suspended; while the waste G0 is not fed from the dryer 113, on the other hand, the waste G0 can be fed from the second storage unit 112. This mode allows the waste G0 to be fed to the gasifier 114 without interruption by feeding the waste G0 from the second storage unit 112, even when the dryer 113 is out of operation, for example, because of maintenance.

The hopper 123A has a considerable capacity, also functioning as a storage unit (hereinafter, occasionally referred to as the "third storage unit") capable of storing the waste G0. For example, the waste G0 may be absent in the second storage unit 112 while the dryer 113 is under maintenance, and in this case the waste G0 can be fed neither from the dryer 113 nor from the second storage unit 112; however, even in such a case, the waste G0 could be fed to the gasifier 114 without interruption for a while by virtue of the waste G0 stored in the hopper 123A.

The waste more than the loading into the gasifier 114 may be temporarily fed to the hopper 123A, for example, because the waste G0 is fed from both the dryer 113 and the second storage unit 112; however, even in such a case, the hopper 123A (third storage unit) can store an excess part of the waste G0.

### (Gasifier)

The gasifier 114 gasifies the waste G0 fed to generate synthetic gas. The gasifier 114 includes a gasification furnace 115 and a reforming furnace 116, and may be a gasifier of any type, as described for the first embodiment. The details of the gasifier 114 and the details of the step performed therein are the same as those in the first embodiment, and description of them is omitted.

### (Organic Substance Generation Unit)

The synthetic gas G1 given by the gasifier 114 is sent to the organic substance generation unit 117. As illustrated in Fig. 4, after being appropriately treated in the post-stage treatment device 118, the synthetic gas G1 can be sent to the organic substance generation unit 117 in normal cases. In the post-stage treatment device 118, impurities contained in the synthetic gas G1 are removed and the synthetic gas G1 is cooled, as appropriate; for example, the synthetic gas G1 can be cooled to 40°C or lower and fed to the organic substance generation unit 117. The post-stage treatment device 118 is described later.

The organic substance generation unit 117 generates an organic substance by bringing the synthetic gas G1 fed into contact with a catalyst such as a microbial catalyst and a metal catalyst. A microbial catalyst is preferred as the catalyst. Use of a microbial catalyst allows an organic substance to be provided at a high yield under low temperature.

A gas-assimilating microorganism is preferably used as a microbial catalyst. If a microbial catalyst is used, the organic substance generation unit 117 includes a fermentation tank (reactor) filled with culture solution containing water and a microbial catalyst. The synthetic gas G1 is fed into the fermentation tank, and converted into an organic substance within the fermentation tank. The configuration of the fermentation tank, the details of the step performed in the fermentation tank, and the details of the organic substance to be generated are as described for the first embodiment.

Examples of the metal catalyst include active metal for hydrogenation or an assembly of active metal for hydrogenation and auxiliary active metal. The active metal for hydrogenation can be, for example, metal known to allow synthesis of ethanol from mixed gas, and examples thereof include alkali metals such as lithium and sodium, elements of Group 7 in the periodic table such as manganese and rhenium, elements of Group 8 in the periodic table such as ruthenium, elements of Group 9 in the periodic table such as cobalt and rhodium, and elements of Group 10 in the periodic table such as nickel and palladium.

These active metals for hydrogenation may be used singly, and two or more thereof may be used in combination. For a further enhanced CO conversion rate and enhanced ethanol selectivity, the active metal for hydrogenation is preferably a combination of rhodium or ruthenium, an alkali metal, and another active metal for hydrogenation, such as a combination of rhodium, manganese, and lithium, and a combination of ruthenium, rhenium, and sodium.

Examples of the auxiliary active metal include titanium, magnesium, and vanadium. The auxiliary active metal supported in addition to the active metal for hydrogenation can enhance, for example, the CO conversion rate and the ethanol selectivity.

The metal catalyst is preferably a rhodium-based catalyst. An additional metal catalyst other than rhodium-based catalysts may be used in combination with the rhodium-based catalyst. Examples of the additional metal catalyst include a catalyst in which copper alone or copper and a non-copper transition metal is/are supported on a support.

If a metal catalyst is used, the organic substance generation unit 117 likewise includes a reactor, and an organic substance can be generated by bringing the synthetic gas G1 into contact with the metal catalyst within the reactor. The temperature within the reactor can be maintained, for example, within 100 to 400°C, preferably within 100 to 300°C.

### (Moisture-Measuring Device)

The moisture-measuring device 121 measures the moisture content of the waste G0. The moisture-measuring device 121 is not limited and may be any moisture-measuring device that can measure the moisture content of the waste G0; for example, the moisture-measuring device 121 may be a noncontact moisture meter such as an optical moisture meter, or a contact moisture meter such as an electric moisture meter. Examples of the optical moisture meter include an infrared moisture meter. Examples of the electric moisture meter include a moisture meter that measures moisture by measuring electrical resistance or electrical capacity. Alternatively, the moisture-measuring device 121 may be a moisture meter that measures moisture by drying a predetermined amount of a sample collected.

The moisture content of the waste G may be determined from a measurement at one point, and the mean for a plurality of points may be used as the moisture content of the waste.

It is preferable for the moisture-measuring device 121 to include at least one of a protective instrument and a washing instrument. The washing instrument can be, for example, any washing instrument that can apply washing solution such as water onto the detector of the moisture-measuring device 121, and the washing instrument can spray washing solution onto the detector. The washing instrument may be a pneumatic washing instrument, for example, a washing instrument that washes the detector by blowing compressed air onto the detector.

Examples of the protective instrument include a covering material to be disposed to cover the detector of the moisture-measuring device 121. Examples of the covering material include a glass covering material and a plastic covering material. The protective instrument and the washing instrument may be used in combination, and a configuration may be employed in which the washing instrument applies washing solution or blow compressed air onto the protective instrument.

Inclusion of at least one of the protective instrument and the washing instrument allows the moisture-measuring device 121 to accurately measure the moisture content with ease by preventing the contamination with the waste G.

The moisture-measuring device 121 is disposed in the pre-stage of the dryer 113, and measures the moisture of the waste G0 before being dried by the dryer 113. Specifically, the moisture-measuring device 121 can measure the moisture content of the waste G0 taken out of the first storage unit 111 by the crane 122 as a take-out device.

The position where the moisture-measuring device 121 is to be disposed is not limited; for example, the moisture-measuring device 121 may be attached to the crane 122, and this configuration allows easy measurement of the moisture content of the waste G0 held and taken out by the crane 122. If the moisture-measuring device 121 is a contact moisture meter, for example, the moisture-measuring device 121 is provided in the inner surface side of the holding section 122A in contact with the waste G0 as illustrated in Fig. 5, and this configuration allows measurement of the moisture of the waste G0 held by the holding section 122A.

If the moisture-measuring device 121 is a noncontact moisture meter, the moisture-measuring device 121 can be disposed at a position where the moisture content of the waste G0 held by the crane 122 can be detected; for example, if the moisture-measuring device 121 is an optical moisture meter, the moisture-measuring device 121 can be disposed at a position where light from the waste G0 held by the crane 122 can be detected. Specifically, the moisture-measuring device 121 can be provided, for example, at a part of the holding section 122A connecting to the hanging section 122B or the vicinity thereof, or on the hanging section 122B.

If the moisture-measuring device 121 is a noncontact moisture meter, the moisture-measuring device 121 does not need to be attached to the crane 122, and may be provided on a ceiling or wall of a building in which the first storage unit 111 is to be provided, or hung from the ceiling.

In the present embodiment, the direction of feeding of the waste G0 taken out of the first storage unit 111 by the crane 122 is switched in a manner depending on the result of measurement by the moisture-measuring device 121. Specifically, if the moisture content determined by the moisture-measuring device 121 is equal to or higher than a threshold X specified in advance, it is determined that the waste G0 contains a large amount of moisture, and the waste G0 is directly transferred by the crane 122 to be fed to the dryer 113. Thereafter, the waste G0 determined to have a high moisture content is dried by the dryer 113 and then fed to the gasifier 114.

On the other hand, if the moisture content determined by the moisture-measuring device 121 is lower than a threshold X specified in advance, it is determined that the waste G0 contains a small amount of moisture, and the waste G0 is directly transferred by the crane 122 to the second storage unit 112, and stored in the second storage unit 112. Thereafter, the waste G0 is fed from the second storage unit 112 to the gasifier 114 via the dust feeder 123. That is, the waste G0 whose determined moisture content is lower than a threshold X is fed to the gasifier 114 without passing through the dryer 113.

Thus, in the present embodiment, the waste G0 whose moisture content is equal to or higher than a threshold X is dried by the dryer 113 to lower the moisture content, and then fed to the gasifier 114; as a result, the waste G0 to be fed to the gasifier 114 always has such a low moisture content, and the unevenness of the moisture content can be reduced. Accordingly, the variation of the generations of hydrogen and carbon monoxide per unit time is reduced in the gasifier 114, and thus the variation of feed rates of hydrogen and carbon monoxide per unit time to the organic substance generation unit 117 is lowered, too.

Therefore, even if the amount of the microbial catalyst in the organic substance generation unit 117 is increased, shortage of feed rates of hydrogen or carbon monoxide due to the variation of the generations of hydrogen and carbon monoxide is less likely to be caused, and the resulting fewer deaths of the microbial catalyst allow efficient generation of an organic substance such as ethanol by using the microbial catalyst.

In addition, the gasifier 114 can efficiently generate the synthetic gas G1 by virtue of the stably low moisture content of the waste G0 to be fed to the gasifier 114.

The waste G0 is dried by the dryer 113 only when it is determined that the moisture content is equal to or higher than a threshold X. Accordingly, a lower energy load is provided and this contributes to achievement of efficient generation of an organic substance. Moreover, the dryer 113 can be intermittently operated because the dryer 113 is only required to dry a part of the waste G0, which leads to less frequent machine trouble in the dryer 113 and easiness in maintenance or the like while the dryer 113 is out of operation.

Even when feeding of the waste G0 from the dryer 113 to the gasifier 114 is suspended during the dryer 113 is under maintenance, the waste G0 can be fed to the gasifier 114 without interruption by feeding the waste G0 stored in the second storage unit 112 to the gasifier 114, which facilitates continuous operation of the organic substance production device 110.

The moisture collected from the dryer 113 can be reused in the organic substance production device 110. For example, the moisture can be fed to and used for a water scrubber described later. Reuse of the moisture collected from the dryer 113 allows waste water generated in association with drying to be effectively used without discarding, which is preferable in view of environmental conservation.

In the present embodiment, the above-mentioned threshold X can be set to a value, for example, selected from the range of 10 to 35% by mass. If the threshold X is set to a value of 35% by mass or lower, then the waste G0 is to be dried when having a moisture content equal to or lower than the threshold X, and hence the unevenness of the moisture content of the waste G0 to be fed to the gasifier 114 can be sufficiently reduced.

The spontaneous ignition of the waste G0 dried by the dryer 113 can be prevented by setting the threshold X to a value of 10% by mass or higher and drying the waste G0 having a moisture content equal to or higher than the threshold X by the dryer 113. In addition, if the threshold X is set to a value of 10% by mass or higher, a larger amount of the waste G0 is to be dried, resulting in less frequent occurrence of troubles such as excessively high energy loads and difficulty in maintenance due to the excessively long operation time of the dryer 113.

From such viewpoints, the threshold X can be set preferably within the range of 20 to 30% by mass, more preferably within the range of 23 to 27% by mass, and the threshold X is most preferably set to 25% by mass.

The organic substance production device 110 may be provided with a controller (not shown). The controller can be configured with a personal computer or the like. The controller is a unit configured to control the action of the first crane 122, and can control take-out of the waste G0 from the first storage unit 111 and feeding of the waste G0 taken out to the dryer 113 or the second storage unit 112 by the crane 122.

To the controller, measurements of the moisture content from the moisture-measuring device 121 are inputted, and whether each measurement is equal to or higher than the threshold X is determined. The controller controls the action of the first crane 122 on the basis of the determination result. Specifically, if the moisture content is lower than the threshold X, the first crane 122 is forced to transfer the waste G0 held thereby to feed to the second storage unit 112. On the other hand, if the moisture content is equal to or higher than the threshold X, the first crane 122 is forced to transfer the waste G0 held thereby to feed to the dryer 113.

However, the controller does not need to be included. The organic substance production device 110 may be provided with an operation panel to operate the crane 122, and the action of the crane 122 may be controlled by input from the operation panel.

Similarly, other actions such as feeding of the waste G0 from the dryer 113 to the gasifier and feeding of the waste G0 from the second storage unit 112 to the gasifier 114 by the second crane 127 may be controlled by the controller, or controlled by input from an operation panel, or controlled by another means.

### (Post-stage treatment device)

In the post-stage treatment device 118, impurities contained in the synthetic gas G1 are removed and the temperature of the synthetic gas G1 is adjusted, as appropriate. If a microbial catalyst is used as the catalyst in the organic substance generation unit 117 described later, the synthetic gas G1 can be cooled to 40°C or lower, preferably to 38°C or lower, and fed to the organic substance generation unit 117. If a metal catalyst is used as the catalyst in the organic substance generation unit 117, the synthetic gas G1 can be fed to the organic substance generation unit 117 after the temperature of the synthetic gas G1 is adjusted to a temperature at which the metal catalyst can be activated, for example, to 100 to 400°C, preferably to about 100 to 300°C.

Examples of the post-stage treatment device 118 include the processor shown for the first embodiment. One processor may be used singly, and two or more processors may be used in combination.

It is preferable for the post-stage treatment device 118 to include at least a heat exchanger, a gas-cooling tower, a filter dust collector, and a water scrubber in the presented order from the pre-stage. In particular, if a microbial catalyst is used as the catalyst, inclusion of them is preferred.

The configurations of the heat exchanger, gas-cooling tower, filter dust collector, and water scrubber and the steps performed therein are as described for the first embodiment.

The synthetic gas discharged from the water scrubber may be appropriately passed through one or more of the above processors other than the heat exchanger, the gas-cooling tower, the filter dust collector, and the water scrubber for purification, cooling, or any other processing of the synthetic gas.

Although a configuration in which the heat exchanger, the gas-cooling tower, the filter dust collector, and the water scrubber are all provided in the post-stage of the gasifier 114 has been described hereinabove, some or all of them may be omitted. Even if the water scrubber is omitted, for example, another cooling device can be provided in the post-stage to cool the synthetic gas G1, for example, to 40°C or lower and feed the synthetic gas G1 to the organic substance generation unit 117, in a case where the catalyst is a microbial catalyst. At least one of the heat exchanger, the gas-cooling tower, and the filter dust collector may also be omitted, and the purification, cooling, or any other processing of the synthetic gas can be performed only with processors other than the heat exchanger, the gas-cooling tower, the filter dust collector, and the water scrubber.

### (Purification Device)

The organic substance production device 110 may include a purification device (not shown) for purifying an organic substance produced by the organic substance generation unit 117.

If a metal catalyst is used to generate ethanol or the like as the target product, for example, a product containing not only ethanol but also organic substances other than ethanol such as acetaldehyde and acetic acid is formed in normal cases. Accordingly, an organic substance (e.g., ethanol) as the target product may be purified by using a known purification device such as a distillation device to obtain the target product.

### (Separator)

While an organic substance is generated, for example, with a microbial catalyst to give an organic substance solution in the organic substance generation unit 117 as described above, the organic substance production device 110 may include a separator (not shown) configured to separate at least water from the organic substance solution.

Examples of the separator include a solid-liquid separator, a distillation device, and a separation membrane, and use of a solid-liquid separator and a distillation device in combination is preferred.

It should be noted that the separator may be omitted, for example, if the organic substance produced in the organic substance generation unit 117 does not need purification or if separation of water from the organic substance solution is not needed.

The separation step performed by using a solid-liquid separator and a distillation device in combination is the same as that in the first embodiment.

In the solid-liquid separator, the organic substance solution given in the organic substance generation unit 117 can be separated into a solid component primarily containing the microorganism and a liquid component containing the organic substance, as described above; here, the solid component may be fed as the waste G0 to the gasifier 114. This enables effective use of waste formed in generating an organic substance. At that time, the moisture content of the solid component is measured, as described above; the solid component can be dried by the dryer 113 and then fed to the gasifier 114 if the moisture content is equal to or higher than the threshold, and the solid component can be fed to the gasifier 114 without passing through the dryer 113 if the moisture content is lower than the threshold.

As with the case of the first embodiment, the water separated in the separator is preferably reused, and, for example, can be fed to the gas-cooling tower of the post-stage treatment device 18 described later and used for water spray in the gas-cooling tower.

### <Fourth Embodiment>

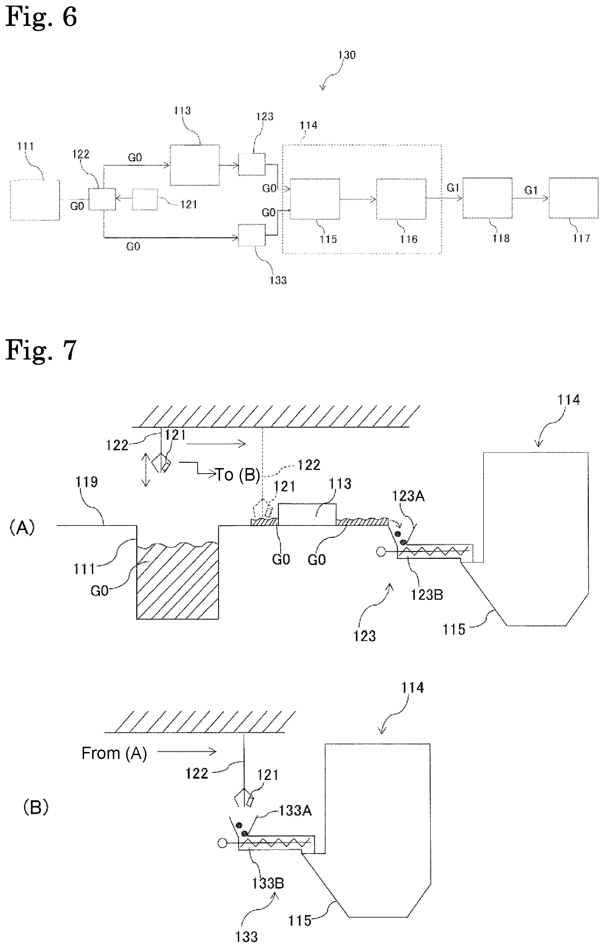

Fig. 6 shows a block diagram illustrating an organic substance production device according to the fourth embodiment of the present invention. Fig. 7 shows a schematic diagram illustrating in detail a part of the organic substance production device 130 according to the fourth embodiment.

The organic substance production device 130 according to the fourth embodiment differs from that according to the third embodiment in that the second storage unit 112 as a waste pit is omitted, and that a second dust feeder 133 is included in addition to the above-described dust feeder 123 (hereinafter, occasionally referred to as the "first dust feeder 123" for convenience) as a dust feeder to feed the waste G0 to the gasifier 114. In the fourth embodiment, the second crane 127 is also omitted since the second storage unit 112 as a waste pit is omitted. While the second storage unit 112 as a waste pit is omitted in the fourth embodiment, a hopper 133A of the second dust feeder 133 can serve as a second storage unit as described later.

The organic substance production device and production method for an organic substance according to the fourth embodiment of the present invention will now be described in detail with reference to Figs. 6 and 7. In the following description, a component having the same configuration as the corresponding component in the third embodiment is provided with the same reference sign, and description thereof is omitted.

As illustrated in Fig. 7(B), for example, the second dust feeder 133 includes a hopper 133A and a feed screw 133B, and moves the waste G0 loaded into the hopper 133A by rotating the feed screw 133B, thereby feeding the waste G0 to a gasification furnace 115. Thus, two dust feeders (the first and second dust feeders 123, 133) are connected to the gasifier 114, and the waste is fed by the two dust feeders.

In the present embodiment, the moisture content of the waste G0 taken out by the crane 122 is measured by the moisture-measuring device 121, with the waste G0 held by the crane 122, as in the third embodiment. If the determined moisture content of the waste G0 is equal to or higher than a threshold X, the waste G0 held by the crane 122 is directly fed to the dryer 113, dried by the dryer 113, and then fed to the gasifier 114 via the first dust feeder 123.

On the other hand, if the determined moisture content of the waste G0 is lower than a threshold X, the waste G0 taken out by the crane 122 is directly fed to the second dust feeder 133 by the crane 122 without passing through the dryer 113. Then, the waste G0 not dried by the dryer 113 is fed from the second dust feeder 133 to the gasifier 114.

Here, the waste G0 may be fed from the first and second dust feeders 123, 133 to the gasifier 114 in any mode; for example, both the first and second dust feeders 123, 133 may be driven to feed the waste G0 from both the first and second dust feeders 123, 133, but it is preferable that the waste G0 be fed from only one of the first and second dust feeders 123, 133.

Accordingly, it is preferable to suspend feeding of the waste G0 from the second dust feeder 133 to the gasifier 114 while the waste G0 is fed from the first dust feeder 123 to the gasifier 14. Otherwise, it is preferable to suspend feeding of the waste G0 from the first dust feeder 123 to the gasifier 114 while the waste G0 is fed from the second dust feeder 133.

The hopper 133A in the second dust feeder 133 has a considerable capacity, hence being also capable of serving as a storage unit to store the waste G0. That is, the hopper 133A functions as a storage unit (second storage unit) as an alternative of the omitted waste pit. Thus, while the waste G0 cannot be fed from the first dust feeder 123 to the gasifier 114, for example, because of maintenance of the dryer 113, the waste G0 can be constantly fed to the gasifier 114 without interruption by feeding the waste G0 stored in advance in the hopper 133A of the second dust feeder 133 to the gasifier 114.

The hopper 133A can store the waste G0 in advance, while feeding of the waste G0 from the second dust feeder 133 to the gasifier 114 is suspended, for example.

Likewise, the hopper 123A of the first dust feeder 123 functions as a storage unit (third storage unit). Thus, the waste G0 fed from the dryer 113 to the first dust feeder 123 can be stored in the hopper 123A, while feeding of the waste G0 from the first dust feeder 123 to the gasifier 114 is suspended, for example. Even when feeding of the waste G0 from the dryer 113 is suspended, the waste G0 stored in the hopper 123A could be fed to the gasifier 114 by the first dust feeder 123 for a while.

Thus, in the present embodiment, even when feeding of the waste G0 from the dryer 113 is suspended for a while, for example, because of maintenance, the waste G0 can be fed to the gasifier 114 without interruption by using the hoppers 123A, 133A as storage units, and the continuous operation of the organic substance production device 130 is not disturbed.

### <Modifications of Third and Fourth Embodiments>

The thus-described organic substance production device and production method for an organic substance as illustrated with the third and fourth embodiments are an example of the present invention, and the present invention is not limited to the configurations of those embodiments; any improvement and modification can be made without departing from the spirit of the present invention, and another component may be added as appropriate. In addition, the configurations of the first to fourth embodiments may be appropriately combined.

A mode in which two cranes are provided has been shown for the third embodiment; however, for example, one crane may be provided, or three or more cranes may be provided.

A mode in which the first storage unit 111 and the second storage unit 112 are disposed at positions distant from each other has been shown for the third embodiment; however, the first storage unit 111 and the second storage unit 112 may be disposed at positions adjacent to each other. If the first storage unit 111 and the second storage unit 112 are adjacent to each other, it is easier to transfer the waste from the first storage unit 111 to the second storage unit 112, which permits reduction of the number of cranes.

A mode in which one crane is provided has been shown for the fourth embodiment; however, two or more cranes may be provided, similarly.

The crane 122 has been shown as a means for taking out the waste G0 from the first storage unit 111 for each of the third and fourth embodiments; however, the waste G0 may be taken out of the first storage unit 111 by a means other than the crane 122. Examples of the means include a conveyor belt and a hydraulic excavator.

A mode in which one dust feeder is used has been shown for the third embodiment; however, two dust feeders may be provided so that the waste G0 from the dryer 113 and the waste G0 from the second storage unit 112 can be fed to the gasifier 114 via different dust feeders.

Similarly, a mode in which two dust feeders are provided has been shown for the fourth embodiment; however, one dust feeder may be used, and in this case the waste G0 dried by the dryer 113 and the waste G0 without passing through the dryer 113 may be fed to the same dust feeder and fed together to the gasifier 114 via the one dust feeder.

A crane or a combination of a crane and a dust feeder has been shown as a means for feeding the waste G0 stored in the first storage unit 111 to the dryer 113, the second storage unit 112, or the gasifier 114 (feeder) for the third and fourth embodiments; however, the waste G0 may be fed by a means other than them. For example, a conveyor other than them may be used whose power source is electricity, gas such as air and nitrogen, or steam, and for example, a conveyor belt or a hopper may be used. The feeder may be a combination of any two or more devices selected from the group consisting of a crane, a dust feeder, a conveyor belt, a hopper, and any other device.

A combination of the crane 127 and the dust feeder 123 has been shown as a means for feeding the waste G0 stored in the second storage unit 112 to the gasifier 114 for the third embodiment; however, a conveyor other than them may be used whose power source is electricity, gas such as air and nitrogen, or steam, similarly, and for example, a conveyor belt or a hopper may be used. A combination of any two or more devices selected from the group consisting of a crane, a dust feeder, a conveyor belt, a hopper, and any other device may be used. Accordingly, at least one of the crane and the dust feeder may be omitted as appropriate in the third and fourth embodiments.

In the third embodiment, the waste G0 dried by the dryer 113 is fed to the gasifier 114 via the dust feeder 123 without passing through the second storage unit 112; however, the waste G0 dried by the dryer 113 may be fed to the second storage unit 112. In such a mode, the second storage unit 112 stores both the waste G0 having a moisture content lower than the threshold X and fed without passing through the dryer 113 and the waste G0 dried by the dryer 113 and having a reduced moisture content. Then, the waste G0 mixed, as necessary, by the crane 122 or 127 or the like in the second storage unit 112 is fed to the gasifier 114 via the dust feeder 123.

In the third and fourth embodiments, the moisture content of the waste G0 taken out of the first storage unit 111 by the take-out device configured with a crane is measured; however, the moisture-measuring device 121 may measure the moisture content in any mode that allows measurement of the pre-drying moisture content of the waste G0. For example, the moisture content of the waste G0 stored in the first storage unit 111 may be measured. The moisture content of the waste G0 after being taken out of the first storage unit 111 by a take-out device such as a crane may be measured while the waste G0 is conveyed by a conveyor other than cranes.

Alternatively, the moisture content of the waste G0 before being stored in the first storage unit 111 may be measured. In this case, in the third embodiment, for example, the waste G0 having a moisture content equal to or higher than the threshold can be directly fed to the first storage unit 111 and stored therein, and the waste G0 having a moisture content lower than the threshold can be fed to the second storage unit 112 and stored therein. Thereafter, the waste G0 stored in the first storage unit 111 can be fed to the dryer 113, dried by the dryer 113, and then fed to the gasifier 114 via a conveyor such as a dust feeder. The waste G0 stored in the second storage unit 112 can be fed to the gasifier 14 via a conveyor such as a crane and a dust feeder without passing through the dryer 111.

Further, the first storage unit 111 may be omitted as appropriate; on the basis of a result of measurement of the moisture of the waste G0 by the moisture-measuring device, the waste G0 may be fed to the dryer 113 or the like without being stored in the first storage unit 111.

In the gasifier 114, two or more types of waste may be mixed and combusted, as described above; for example, municipal solid waste (MSW) mixed with waste having a low moisture content such as plastic or waste having a high fuel efficiency such as wood chips may be combusted.

In a mode shown in the above descriptions of the third and fourth embodiments, the moisture content of the waste G0 is measured, and the waste G0 whose determined moisture content is equal to or higher than the threshold is fed to the dryer 113 whereas the waste G0 whose determined moisture content is lower than the threshold is fed to the gasifier 114 without passing through the dryer 113. However, measurement of the moisture content of the waste G0 is not necessarily needed for some waste types, and plastic, wood chips, and the like mentioned above may be fed to the gasifier 114 with neither measurement of the moisture content nor drying by the dryer 113.

Specifically, in the third and fourth embodiments, two types of waste, the waste G0 dried by the dryer 113 and the waste G0 that has not been passed through the dryer 113 because the determined moisture content is lower than the threshold, are fed to the gasifier 114; however, specific types of waste such as plastic and wood chips may be fed in addition to them to the gasifier 114 without being subjected to moisture measurement and drying by the dryer 113.

In the third and fourth embodiments, a storage unit such as a waste pit for storing specific types of waste such as plastic and wood chips may be separately provided.

### Reference Signs List

10, 30, 110, 130: organic substance production device
11, 111: storage unit (first storage unit)
12, 112: storage unit (second storage unit)
13, 113: dryer
14, 114: gasifier
15, 115: gasification furnace
16, 116: reforming furnace
17, 117: organic substance generation unit
18, 118: post-stage treatment device
19, 119: platform
22: crane (waste feeder)
23: dust feeder (waste feeder)
32: crane (waste feeder)
121: moisture-measuring device
122: first crane
123: first dust feeder
127: second crane
133: second dust feeder
G0: waste
G1: synthetic gas

## Claims

1. A production method for an organic substance, the production method comprising:
a step of feeding waste to a dryer;
a step of drying the waste by the dryer;
a step of feeding the waste dried by the dryer to a gasifier;
a step of gasifying the waste by the gasifier to generate synthetic gas; and
a step of bringing the synthetic gas into contact with a catalyst to generate an organic substance.

2. The production method for an organic substance according to claim 1, the production method further comprising:
a step of receiving waste in a first storage unit, wherein
the waste stored in the first storage unit is fed to the dryer.

3. The production method for an organic substance according to claim 1 or 2, the production method comprising:
a step of feeding waste dried by the dryer to a second storage unit, wherein
the waste stored in the second storage unit is fed to the gasifier.

4. The production method for an organic substance according to claim 3, the production method comprising:
a step of mixing the waste stored in the second storage unit.

5. The production method for an organic substance according to any one of claims 1 to 4, wherein the waste is dried to reach a moisture content of 5% by mass or more and 30% by mass or less and fed to the gasifier.

6. The production method for an organic substance according to any one of claims 1 to 5, the production method comprising:
a step of measuring the moisture content of at least one of the waste before being dried by the dryer and the waste dried by the dryer.

7. The production method for an organic substance according to any one of claims 1 to 5, the production method further comprising:
a step of measuring the moisture content of waste, wherein
waste whose determined moisture content is equal to or higher than a threshold is fed to the dryer.

8. The production method for an organic substance according to claim 7, wherein waste whose determined moisture content is lower than the threshold is fed to the gasifier without passing through the dryer.

9. The production method for an organic substance according to claim 7 or 8, wherein the threshold is set within a range of 10 to 35% by mass.

10. The production method for an organic substance according to any one of claims 7 to 9, the production method comprising:
a step of feeding waste whose determined moisture content is lower than the threshold to a second storage unit to store the waste in the second storage unit; and
a step of feeding the waste stored in the second storage unit to the gasifier.

11. The production method for an organic substance according to any one of claims 6 to 10, wherein waste stored in a first storage unit is taken out and the moisture content of the waste is measured.

12. The production method for an organic substance according to claim 11, wherein the waste stored in the first storage unit is taken out by a crane and held by the crane and the moisture content of the waste held is measured.

13. The production method for an organic substance according to claim 12, wherein the crane transfers waste whose determined moisture content is equal to or higher than a threshold to feed to the dryer, and transfers waste whose determined moisture content is lower than the threshold to feed to the gasifier without passing through the dryer.

14. The production method for an organic substance according to any one of claims 6 to 13, wherein a moisture-measuring device configured to measure the moisture content of waste comprises a protective instrument or a washing instrument.

15. The production method for an organic substance according to any one of claims 1 to 14, wherein the organic substance comprises ethanol.

16. The production method for an organic substance according to any one of claims 1 to 15, wherein the catalyst is a microbial catalyst.

17. An organic substance production device, comprising:
a dryer configured to dry waste;
a gasifier configured to gasify waste to generate synthetic gas; and
an organic substance generation unit configured to bring the synthetic gas into contact with a catalyst to generate an organic substance, wherein
the organic substance production device is capable of feeding waste dried by the dryer to the gasifier.

18. The organic substance production device according to claim 17, further comprising:
a first storage unit configured to receive waste; and
a waste feeder capable of feeding waste stored in the first storage unit to the dryer and capable of feeding waste dried by the dryer to the gasifier.

19. The organic substance production device according to claim 18, further comprising:
a second storage unit, wherein
the waste feeder is capable of feeding waste dried by the dryer to the second storage unit and capable of feeding waste stored in the second storage unit to the gasifier.

20. The organic substance production device according to claim 19, wherein
the waste feeder is capable of mixing waste stored in the second storage unit, or
the organic substance production device further comprises a mixer configured to mix waste stored in the second storage unit.

21. The organic substance production device according to any one of claims 18 to 20, wherein
the waste feeder feeds waste dried to reach a moisture content of 5% by mass or more and 30% by mass or less to the gasifier.

22. The organic substance production device according to any one of claims 17 to 21, comprising:
a moisture-measuring device configured to measure the moisture content of at least one of waste before being dried by the dryer and waste dried by the dryer.

23. The organic substance production device according to any one of claims 17 to 21, further comprising:
a moisture-measuring device configured to measure the moisture content of waste, wherein
the organic substance production device is capable of feeding waste whose moisture content has been determined to the dryer.

24. The organic substance production device according to claim 23, being capable of feeding waste whose moisture content has been determined to the gasifier without passing through the dryer.

25. The organic substance production device according to claim 23 or 24, wherein waste whose determined moisture content is equal to or higher than a threshold is fed to the dryer, and waste whose determined moisture content is lower than the threshold is fed to the gasifier without passing through the dryer.

26. The organic substance production device according to claim 25, wherein the threshold is set within a range of 10 to 35% by mass.

27. The organic substance production device according to any one of claims 23 to 26, further comprising:
a second storage unit configured to be fed with waste whose moisture content has been determined and store the waste, wherein
the organic substance production device is capable of feeding waste stored in the second storage unit to the gasifier.

28. The organic substance production device according to any one of claims 22 to 27, comprising:
a first storage unit configured to store waste; and
a take-out device configured to take out waste stored in the first storage unit, wherein
the moisture-measuring device measures the moisture content of waste taken out by the take-out device.

29. The organic substance production device according to claim 28, wherein
the take-out device is a crane, and
the moisture-measuring device measures the moisture content of waste take out and held by the crane.

30. The organic substance production device according to claim 29, wherein
the crane is capable of transferring waste whose moisture content has been determined to feed to the dryer or feed to the gasifier without passing through the dryer.

31. The organic substance production device according to any one of claims 22 to 30, wherein
the moisture-measuring device comprises a protective instrument or a washing instrument.

32. The organic substance production device according to any one of claims 17 to 31, wherein the organic substance comprises ethanol.

33. The organic substance production device according to any one of claims 17 to 32, wherein the catalyst is a microbial catalyst.
